**Europäisches Patentamt**

**European Patent Office**

**Office européen des brevets**

(19)

(11) Numéro de publication : **0 297 010 B1**

(12) # FASCICULE DE BREVET EUROPEEN

(45) Date de publication du fascicule du brevet :
**21.08.91 Bulletin 91/34**

(21) Numéro de dépôt : **88440049.0**

(22) Date de dépôt : **20.06.88**

(51) Int. Cl.⁵ : **A61L 2/06, A23L 3/10**

(54) **Autoclave.**

(30) Priorité : **22.06.87 FR 8708848**

(43) Date de publication de la demande :
**28.12.88 Bulletin 88/52**

(45) Mention de la délivrance du brevet :
**21.08.91 Bulletin 91/34**

(84) Etats contractants désignés :
**AT BE CH DE ES FR GB GR IT LI LU NL SE**

(56) Documents cités :
**EP-A- 0 138 688**
**DE-A- 1 946 090**
**FR-A- 2 201 900**
**FR-A- 2 403 801**
**GB-A- 2 147 823**

(73) Titulaire : **Camu, Patrice**
**5, rue des Pâquerettes**
**F-67115 Plobsheim (FR)**

(72) Inventeur : **Camu, Patrice**
**5, rue des Pâquerettes**
**F-67115 Plobsheim (FR)**

(74) Mandataire : **Aubertin, François**
**Cabinet Lepage & Aubertin Innovations et**
**Prestations 4, rue de Haguenau**
**F-67000 Strasbourg (FR)**

## Description

L'invention concerne un autoclave à axe horizontal pour la stérilisation par injection de vapeur comprenant une enceinte équipée d'une porte de chargement étanche, une alimentation en vapeur, une alimentation en air comprimé, un ensemble moteur-ventilateur pour accélérer le mélange air-vapeur, une purge avec soupape de sécurité pour chasser l'air ambiant, une sortie des condensats reliée à un circuit de refroidissement fermé pourvu d'une arrivée d'eau froide extérieure et un échangeur de chaleur pour accélérer le processus de refroidissement.

Le cycle de stérilisation dans de tels appareils met en application des lois de thermodynamique associant la température et la pression. Aussi, lors de la phase de refroidissement, doit-on compenser la chute de pression de la vapeur contenue dans l'enceinte par l'injection d'un gaz de pression supérieure, généralement de l'air comprimé, afin de maintenir l'intérieur de l'enceinte à pression constante.

Cette technique présente deux inconvénients majeurs vis-à-vis du cycle de stérilisation, au cours duquel la pression doit d'abord rester constante puis progressivement descendre à la pression atmosphérique au moment de l'ouverture de l'autoclave.

Le premier inconvénient est dû au fait que l'air comprimé est généralement prélevé sur un réseau de distribution à température ambiante, de l'ordre de 20 degrés Celsius environ. L'injection de cet air comprimé relativement froid par rapport à la vapeur (120 degrés Celsius environ) refroidit cette vapeur, faisant parallèlement baisser sa pression, en sorte que pour rétablir la pression voulue, il faut injecter davantage d'air comprimé.

Le second inconvénient est lié au fait que l'air comprimé nuit à l'homogénéité de la température à l'intérieur de l'autoclave, d'autant plus que cet air comprimé est relativement froid par rapport à la vapeur. Pour y remédier, on fait généralement appel à des moyens connus de brassage du mélange gaz-vapeur, le plus souvent des ventilateurs.

Ces inconvénients ont une influence directe sur les produits en cours de stérilisation, en ce sens que l'homogénéité de la température dans la masse des récipients contenant les produits à stériliser, est difficilement réalisable même avec ces moyens de brassage énergique.

Les expérimentations ont démontré qu'il était difficile, voire impossible, à la chaleur de traverser un bloc ou "pack" de récipients pleins empilés les uns sur les autres en quinconce dans des paniers, même avec des intercalaires plats en polypropylène de deux ou trois millimètres d'épaisseur. On constate que l'on chauffe d'abord le pourtour de ce bloc, puis progressivement, les récipients installés à l'intérieur, ce qui allonge le cycle de stérilisation et entraîne parallèlement une durée de chauffe plus longue des récipients extérieurs, avec pour conséquence une augmentation de la pression dans lesdits récipients extérieurs.

Ces écarts de pression, conséquence directe des écarts de température, rendent la régulation de la pression dans l'autoclave très difficile à ajuster aux pressions existant dans chaque récipient de conservation.

La régulation de la surpression est difficilement maîtrisable par l'injection du gaz ou air comprimé qui a un coefficient de dilatation considérable en un court laps de temps, puisqu'il passe de 20 degrés Celsius environ à plus de cent degrés Celsius à l'intérieur de l'autoclave. Or, les surpressions ponctuelles sont particulièrement néfastes pour les nouveaux récipients de conservation en matière plastique semi-rigide scellés par thermosoudage et sensibles à de très faibles variations de pression.

La seconde phase dite de refroidissement et de décompression progressive pose des problèmes délicats, en particulier celui du choc thermique, qui doit être évité dans le cas d'utilisation de récipients en verre.

On pallie en général à cet inconvénient en usant de procédés compensatoires par une première injection d'eau froide (à température ambiante) dans l'enceinte en évitant des projections sur les récipients.

Ces procédés sont cependant difficiles à mettre en oeuvre du fait que cette injection d'eau froide entraîne une condensation lente de la vapeur allongeant le temps du processus de refroidissement.

Par recyclage du mélange condensats-eau froide, on peut refroidir les récipients, mais l'instabilité en température de ce mélange entraîne le risque de chocs thermiques au niveau des récipients, et une injection non circonscrite au bloc des récipients, mais généralisée à toute l'enceinte, a pour conséquence une surconsommation d'eau froide.

Par ailleurs, les dispositifs actuels de refroidissement ne permettent pas de descendre en-dessous de la température de l'eau du réseau de distribution, ce qui entraîne, dans le cas de produits de semi-conservation, la nécessité de recourir à un refroidissement ultérieur avant introduction dans la chaîne de froid.

On connaît, par le document FR-A-2403801, d'une part, un procédé de stérilisation et, d'autre part, un autoclave pour la mise en oeuvre dudit procédé. Selon le procédé, la vapeur est introduite dans une chambre pour amener l'atmosphère de celle-ci à la température désirée de stérilisation, cette vapeur étant remplacée à un instant donné par de l'air comprimé qui est chauffé jusqu'à la température de stérilisation. A cet effet, l'autoclave comporte une conduite d'alimentation en air comprimé traversant un filtre et qui est relié à une conduite d'amenée de l'air et de la vapeur débouchant dans la chambre de l'autoclave. Cette conduite d'alimentation en air comprimé traverse, entre un filtre stérile et la conduite d'amenée de l'air et de la vapeur, un échangeur thermique externe de la chambre et chauffé par de la vapeur provenant d'un circuit d'alimentation en vapeur pourvu d'une vanne régulatrice commandée par un régulateur agissant en fonction de la température donnée par un capteur disposé à l'intérieur de la chambre.

Ainsi, pour conférer à l'air comprimé une température correspondante à celle existant dans la chambre, on est obligé de faire appel à une source d'énergie supplémentaire externe et de prévoir des moyens de contrôle et de régulation devant vérifier la température de l'air comprimé par rapport à la température régnant dans la chambre de stérilisation.

Par ailleurs, on connaît, par le document EP-A-0138688, un autoclave et un procédé de stérilisation. Cet autoclave comporte un ensemble de ventilateurs introduisant dans une enceinte un certain volume d'air. A cet air est ajoutée de la vapeur par l'intermédiaire d'une rampe d'injection de vapeur vive en provenance d'une source à température élevée et sous pression. Cette rampe d'injection de vapeur assure une injection de vapeur dans le sens de circulation du mélange air-vapeur, ce qui permet d'obtenir un effet d'entraînement améliorant le débit du mélange air-vapeur.

De plus, l'autoclave comporte un échangeur de chaleur longitudinal situé à la partie supérieure de l'autoclave. Cet échangeur de chaleur alimenté soit de vapeur vive en provenance de la même source que celle alimentant la rampe d'injection citée ci-dessus, soit en eau froide à partir d'une seconde source permettant un refroidissement du fluide circulant dans l'échangeur de chaleur. Le cas échéant, ce dernier peut être remplacé par un dispositif statique pour améliorer l'homogénéisation.

Toutefois, cet autoclave est dépourvu de tout moyen permettant une régulation de la pression à maintenir dans l'autoclave pendant la phase de stérilisation par introduction d'air comprimé.

La présente invention a pour but de remédier à ces inconvénients. L'invention telle qu'elle est caractérisée dans les revendications résout le problème consistant à créer un autoclave à axe horizontal pour la stérilisation par injection de vapeur comprenant une enceinte équipée d'une porte étanche, une alimentation en vapeur, une alimentation en air comprimé, un ensemble moteur-ventilateur pour accélérer le mélange air-vapeur, une purge avec soupape de sécurité pour chasser l'air ambiant, une sortie des condensats reliée à un circuit de refroidissement fermé pourvu d'une arrivée d'eau froide extérieure et un échangeur de chaleur pour récupérer les calories des condensats et pour accélérer le processus de refroidissement et comportant un circuit d'air comprimé logé dans l'enceinte et dont l'air comprimé, canalisé dans ledit circuit dès le début de la stérilisation, est réchauffé par la température régnant dans l'enceinte puis est injecté dans le volume de l'enceinte.

Les avantages obtenus grâce à cette invention consistent essentiellement en ceci que, sans apport d'énergie supplémentaire externe, on réchauffe l'air comprimé, canalisé dans le circuit, pour injecter dans l'enceinte, lorsqu'il y a lieu de réguler la pression dans celle-ci, de l'air comprimé à une température quasi identique à celle régnant dans l'enceinte. De ce fait, on annihile tout choc thermique lors de l'introduction de l'air comprimé même au moment du passage délicat entre l'arrêt de la stérilisation et le refroidissement des produits stérilisés.

L'invention est exposée ci-après plus en détail à l'aide de dessins représentant seulement un mode d'exécution.

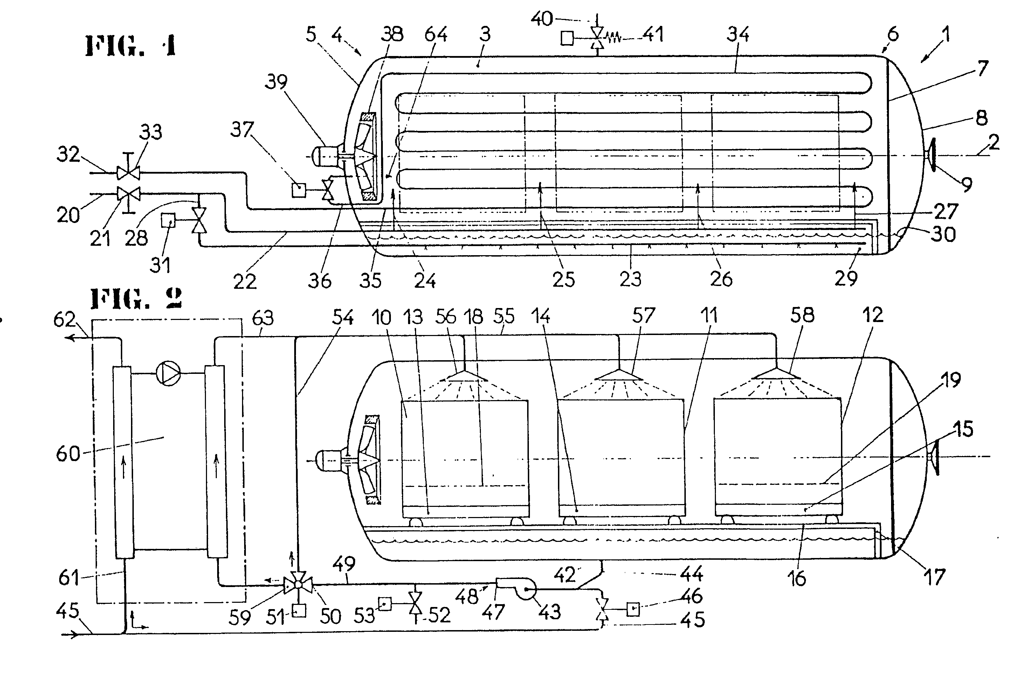

La figure 1 représente schématiquement, en coupe longitudinale, l'autoclave conforme à l'invention avec son alimentation en vapeur d'eau et air comprimé.

La figure 2 représente schématiquement, en coupe longitudinale, l'autoclave pourvu de son circuit de refroidissement et d'arrosage et associé à un groupe de récupération de chaleur.

On se réfère aux deux figures.

L'autoclave de stérilisation 1 à axe horizontal 2 comporte une enceinte de stérilisation cylindrique 3 dont une des extrémités 4 est obturée par un fond 5 et dont l'autre extrémité 6 présente une ouverture 7 pouvant être obturée par une porte 8 étanche à la pression ou dépression régnant dans l'enceinte de stérilisation 3. Bien entendu, cette porte 8 est pourvue de moyens de fermeture hermétique et de sécurité 9.

A travers cette ouverture 7 sont engagés dans l'enceinte 3 ou sont retirés de cette dernière les produits à stériliser. L'aménagement interne de l'enceinte 3 comporte des moyens de stockage des récipients de conservation disposés dans des paniers 10, 11, 12 réalisés de préférence en fil d'acier inoxydable. Ces paniers 10, 11, 12 sont disposés sur des chariots 13, 14, 15 pouvant se déplacer sur des rails de guidage 16 dont une des extrémités 17 est légèrement en retrait par rapport à l'ouverture 7 pour ne pas gêner la manipulation de la porte étanche 8. Avantageusement, on dispose entre deux rangées de récipients des intercalaires 18, 19 formés par exemple d'une tôle perforée ou d'une armature en fil d'acier inoxydable analogue aux paniers 10, 11, 12. Ces intercalaires 18, 19 jouent un rôle important aussi bien dans la phase de montée en température que dans la phase de refroidissement et contribuent à une réduction du temps de cycle de stérilisation.

En effet, on obtient par la présence de ces intercalaires 18, 19 une succession de canaux et passages qui ont pour but de distribuer le fluide de stérilisation entre les rangées de récipients et d'accélérer la montée en température de ceux qui se trouvent stockés dans la zone centrale de chaque rangée, ce fluide de stérilisation étant soumis à une circulation pour assurer le réchauffage simultané de tous les récipients.

L'autoclave de stérilisation 1 comporte une alimentation en vapeur servant de fluide de stérilisation. A cet effet, l'autoclave 1 est relié par une conduite 20 à une source d'alimentation de vapeur. Cette conduite 20 est pourvue d'une vanne d'arrêt 21. En aval de cette vanne d'arrêt 21, on prévoit deux arrivées 22, 23 de vapeur débouchant chacune dans l'enceinte de stérilisation 3 en traversant le fond 5. Ces deux arrivées de vapeur 22, 23 s'étendent pratiquement jusqu'à l'ouverture 7 de ladite enceinte de stérilisation.

La première arrivée de vapeur 22, reliée directement à la vanne d'arrêt 21, comporte une pluralité d'injecteurs 24, 25, 26, 27... à travers lesquels la vapeur est diffusée dans l'enceinte 3 pour assurer la stérilisation des produits mis dans cette dernière.

La seconde arrivée de vapeur 23 mise en dérivation 28 est située à la partie inférieure de l'enceinte 3. Cette seconde arrivée de vapeur 3 est destinée à réchauffer les condensats 29 s'accumulant à ladite partie inférieure de l'enceinte 3. Avantageusement, la seconde arrivée de vapeur 23 est située sous le niveau 30 des condensats 29. Par l'intermédiaire d'une électrovanne 31 on commande l'alimentation en vapeur de la seconde arrivée 23. Ainsi, en réchauffant les condensats 29 pendant la phase de stérilisation, on évite toute perte calorifique tout en évitant un réchauffage des condensats 29 par une source extérieure de chaleur. Il en résulte une grande souplesse d'utilisation dans le cas où l'on doit traiter des produits en vrac dans les paniers 10, 11, 12.

Par ailleurs, l'autoclave de stérilisation 1 est reliée à une conduite d'alimentation en air comprimé 32 pourvue d'une vanne d'arrêt 33.

Selon l'invention, l'enceinte de stérilisation 3 comporte un circuit d'air comprimé 34 disposé longitudinalement le long de la paroi interne de ladite enceinte de stérilisation 3. Avantageusement, ce circuit d'air comprimé 34 a la forme d'un serpentin. L'une de ses extrémités 35 de ce serpentin 34 traverse le fond 5 et est reliée à la vanne d'arrêt 33 de la conduite d'alimentation en air comprimé 32. L'autre extrémité 36 de ce serpentin 34, traversant également le fond 5, comporte une électrovanne de commande 37 et est réintroduite par un retour 64 dans l'enceinte de stérilisation 3 pour permettre d'injecter dans le volume de cette dernière de l'air comprimé. Le volume de ce serpentin 34 est fonction du volume de l'enceinte de stérilisation 3.

Du fait que le serpentin 34 est logé dans l'enceinte de stérilisation 3, l'air comprimé injecté, canalisé par le serpentin 34, comporte une température quasi identique à celle régnant dans l'enceinte de stérilisation 3. Ainsi, sans faire appel à une source de chaleur complémentaire, l'air comprimé introduit dans le serpentin 34 pendant la phase de stérilisation, subit une augmentation de température pour atteindre une température équivalente à celle régnant dans l'enceinte de stérilisation, ce qui évite au moment de l'injection de l'air comprimé des chocs thermiques nuisibles à la stérilisation. Cette augmentation de température provoque une certaine augmentation de la pression de l'air comprimé. Il est hors de doute que l'introduction de cet air comprimé réchauffé facilite considérablement la régulation de la pression à maintenir dans l'enceinte 3 pendant la phase de stérilisation ainsi que le mélange vapeur-air comprimé chaud, améliorant la répartition de la chaleur.

Une sonde de température et un pressostat (non représentés) peuvent gérer le débit de l'air comprimé injecté, ce qui permet de refroidir très progressivement le volume de l'enceinte de stérilisation 3. De cet fait, il n'y a aucun effet de choc de température et de pression, même au moment du passage délicat entre l'arrêt de la chauffe et le refroidissement.

Cette répartition de la chaleur peut être accélérée par la mise en oeuvre d'un ventilateur 38 entraîné par un moteur 39. Cet ensemble ventilateur 38 — moteur 39 est situé du côté du fond 5 de l'enceinte de stérilisation 3. Avantageusement, ce ventilateur 38 peut être de faible puissance et il n'est mis en fonctionnement que pendant des durées relativement courtes.

On dispose à la partie supérieure de l'enceinte de stérilisation 3 et au droit des condensats 29 une purge 40 comportant une soupape de sécurité 41. Cette purge 40 permet de chasser l'air ambiant enfermé dans l'enceinte de stérilisation 3 et remplacé par la vapeur. La fermeture de la purge 40, lors du démarrage du cycle de stérilisation, est avantageusement commandée par une sonde de température d'ambiance de l'enceinte 3 (sonde non représentée), ce qui permet d'assurer un remplissage total de l'enceinte 3 par de la vapeur sans qu'il y ait trace d'air résiduel, à la différence des systèmes connus. Cet avantage est considérable, car il rend possible la succession de cycles rigoureusement identiques.

Conformément à l'invention, l'autoclave de stérilisation 1 comporte un dispositif de recyclage des condensats 29 formés au cours de la phase de stérilisation, ce qui permet d'éviter le choc thermique sur les récipients en assurant le mélange desdits condensats 29 avec une arrivée d'eau froide, ces chocs thermiques risquant de détériorer les récipients, notamment si ces derniers sont en verre.

Le dispositif de recyclage des condensats 29 récupérés dans la partie basse de l'enceinte de stérilisation 3 comporte une sortie 42 des condensats 29 branchée sur une pompe multifonctions 43 par l'intermédiaire d'une conduite 44. Cette pompe multifonctions 43 peut également jouer le rôle de pompe de recyclage, de surpresseur, de régulateur de débit de l'eau ou du mélange eau-condensats 29 et permettre la vidange accélérée en fin d'opération. Sur celle-ci est branchée une arrivée d'eau froide 45 pourvue d'une électrovanne 46. On relie la sortie 47 de cette pompe multifonctions 43 à une des extrémités 48 d'une conduite de refoulement 49 dont l'autre extrémité 50 est reliée à une vanne à trois voies 51. Le cas échéant, cette conduite de refoulement 49 comporte une conduite d'évacuation 52 pourvue d'une électrovanne 53.

Les condensats 29 ou le mélange condensats-eau froide refoulés par la pompe multifonctions 43 à travers la vanne à trois voies 51, sont dirigés par une canalisation 54 vers une rampe 55 pourvue d'arroseurs 56, 57, 58 ... à jets circonscrits au-dessus de chaque panier 10, 11, 12 contenant les récipients séparés par les intercalaires 18, 19, 20.

Le débit d'eau froide peut être contrôlé par l'électrovanne 46 commandée à partir d'une sonde de température (non représentée) gérée par la programmation de l'autoclave 1, ce qui permet de réguler avec précision la descente de température du mélange condensats-eau froide suivant une consigne donnée, soit dégressive en continu, soit par paliers.

Le refroidissement effectif des récipients est accéléré par l'arrosage réalisé par les arroseurs 56, 57, 58 ... de la rampe 55 reproduisant la forme du périmètre défini par les paniers 10, 11, 12 afin de circonscrire chaque zone à refroidir avec un minimum de consommation d'eau et de gagner sur le temps global du cycle de stérilisation.

Pour améliorer les performances de l'autoclave selon l'invention, on peut prévoir de bénéficier des calories véhiculées par les condensats 29 réchauffés par la seconde arrivée de vapeur 23. A cet effet, on relie la troisième voie 59 de la vanne à trois voies 51 à l'entrée d'un échangeur de chaleur 60. Ainsi, il est possible, par la récupération de ces calories, de chauffer un circuit d'eau par exemple d'une installation de chauffage et/ou d'une installation d'eau sanitaire, l'arrivée 61 de l'eau froide étant branchée sur l'arrivée d'eau froide 45 de l'autoclave 1 et l'eau réchauffée 62 étant dirigée vers ladite installation (non représentée). Par voie de conséquence, on refroidit les condensats 29 par récupération des calories, ce qui permet de raccourcir le cycle global de stérilisation par un refroidissement, pouvant être très rapide, des récipients dès que leur température résiduelle le permet. Il en résulte un autre avantage important résidant dans le fait que l'on peut réduire considérablement la consommation d'eau nécessaire pour mener à bien ce refroidissement, cette consommation pouvant être de l'ordre de dix fois inférieure à celle des autoclaves connus. Le cas échéant, on peut remplacer cet échangeur de chaleur 60 par un groupe frigorifique alimenté en fluide frigorifique.

Avantageusement, le fonctionnement de l'autoclave de stérilisation 1 conforme à l'invention peut être le suivant :

• Par l'ouverture 7, on introduit dans l'enceinte de stérilisation, soit en vrac, soit dans les paniers 10, 11, 12, les récipients contenant les produits à stériliser en disposant les intercalaires 18, 19 correctement orientés pour canaliser le fluide de stérilisation formé par la vapeur et l'air et rendre homogène la température dans l'ensemble du volume de l'enceinte de stérilisation 3. Ainsi, chaque récipient subit le même cycle thermique quelle que soit sa position dans ladite enceinte 3. Après fermeture de la porte étanche 8, on met en marche l'ensemble moteur 39—ventilateur 38 puis on injecte via les injecteurs 24, 25, 26, 27 de la première arrivée de vapeur 22 de la vapeur d'eau circulant et se diffusant entre les paniers 10, 11, 12. Simultanément, on ouvre la purge 40 pour permettre l'échappement de l'air ambiant chassé par la vapeur d'eau. Cette purge 40 se fermera automatiquement par la consigne reçue par l'intermédiaire de la sonde de température ambiante de l'enceinte de stérilisation 3 au moyen d'une programmation entièrement automatique, par exemple par microprocesseur ou par micro-ordinateur. Cette programmation surveille également la pression à l'intérieur de l'enceinte et, si nécessaire, commande l'ouverture de la vanne d'arrêt 33 de la conduite d'alimentation de l'air comprimé 32 pour un apport d'air comprimé dans le serpentin 34.

La phase de stérilisation étant atteinte, on procède immédiatement à la phase de refroidissement en mettant en service la pompe multifonctions 43 puisant les condensats 29 éventuellement réchauffés au préalable par une injection de vapeur en provenance de la seconde arrivée de vapeur 23 après ouverture de l'électrovanne 31.

Dans la phase finale de refroidissement, la programmation commande l'ouverture progressive de l'électrovanne 46 permettant l'arrivée de l'eau froide 45. Cette eau froide est mélangée aux condensats 29 et le mélange obtenu entre dans la pompe multifonctions 43. Cette dernière refoule sous pression le mélange eau froide — condensats à travers la vanne à trois voies 51 et la canalisation 54 vers la rampe 55 et les arroseurs 56, 57, 58 ... à jets circonscrits situés au-dessus de chaque panier 10, 11, 12.

Pour éviter une montée excessive du niveau 30 des condensats 29, suite au ruissellement, un détecteur de niveau (non représenté) commande l'électrovanne 53 pour ouvrir la conduite d'évacuation 52.

Par la chute de température, la vapeur d'eau se condense progressivement provoquant dans l'enceinte 3 une chute de pression plus rapide que la chute de la pression renfermée dans les récipients. Pour éviter ce déséquilibre, on remonte la pression dans l'enceinte 3 en actionnant l'électrovanne de commande 37 permettant l'injection d'air comprimé en provenance du serpentin 34 dans l'enceinte 3, cet air comprimé sortant à une température proche de celle régnant dans ladite enceinte 3.

La programmation gère en permanence les conditions de température et pression jusqu'à la décompression et le refroidissement total autorisant l'ouverture de la porte étanche 8. Dans le cas où ledit autoclave 1 est équipé d'un échangeur de chaleur 60, il est possible d'accélérer le processus de refroidissement selon une programmation donnée en absorbant les calories des condensats chauds 29. Le mélange condensats-eau froide refoulé par la pompe multifonctions 43 n'est plus dirigé vers la canalisation 54 mais vers l'échangeur de chaleur 60 abaissant la température de ce mélange. Par une conduite 63, ce mélange refroidi est canalisé vers la rampe 55.

Bien entendu, si l'échangeur de chaleur 60 est remplacé par un groupe frigorifique, le processus de refroidissement est encore plus accéléré et on peut abaisser considérablement la température de l'eau de refroidissement diffusée par les arroseurs 56, 57, 58.

Dans certains cas d'aliments connus, par exemple des plats cuisinés, on pourra abaisser la température de l'enceinte 3 jusqu'à ce que cette température corresponde à celle régnant dans les armoires frigorifiques. Ainsi, il serait possible de transvaser directement les plats depuis l'enceinte 3 dans une armoire frigorifique.

**Revendications**

1. Autoclave à axe horizontal pour la stérilisation par injection de vapeur comprenant une enceinte (3) équipée d'une porte étanche (8), une alimentation en vapeur (20, 22, 23), une alimentation en air comprimé (32, 34, 36, 64), un ensemble moteur (39) — ventilateur (38) pour accélérer le mélange air-vapeur, une purge (40) avec soupape de sécurité (41) pour chasser l'air ambiant, une sortie (42) des condensats (29) reliée à un circuit de refroidissement fermé (43, 49, 54, 55, 56, 57, 58) pourvu d'une arrivée d'eau froide extérieure (45) et un échangeur de chaleur (60) pour récupérer les calories des condensats (29) et pour accélérer le processus de refroidissement, caractérisé en ce qu'il comporte un circuit d'air comprimé (34, 35, 36, 37, 64) logé dans l'enceinte (3) et dont l'air comprimé, canalisé dans ledit circuit (34, 35, 36, 37, 64) dès le début de la stérilisation, est réchauffé par la température régnant dans l'enceinte (3), puis est injecté dans le volume de l'enceinte (3).

2. Autoclave à axe horizontal selon la revendication 1, caractérisé en ce que le circuit d'air comprimé, logé dans l'enceinte (3), comporte un serpentin (34) disposé longitudinalement le long de la paroi interne de l'enceinte (3) dont une des extrémités (35) est reliée à une vanne d'arrêt (33) située sur la conduite d'alimentation en air comprimé (32), et dont l'autre extrémité (36) débouchant vers l'extérieur de l'enceinte (3) en traversant le fond (5) de celle-ci comporte un retour (64) pourvu d'une électrovanne de commande (37) et réintroduit dans l'enceinte (3).

3. Autoclave à axe horizontal selon la revendication 2, caractérisé en ce que le serpentin (34) comporte un volume d'air comprimé proportionnel au volume de l'enceinte (3) et dont le débit de l'air comprimé, injecté dans l'enceinte (3), est géré par une sonde de température et un pressostat logés dans l'enceinte (3) et sollicitant des moyens de programmation entièrement automatique tels que microprocesseur ou micro-ordinateur.

4. Autoclave à axe horizontal selon la revendication 1, caractérisé en ce que l'alimentation en vapeur (20), pourvue d'une vanne d'arrêt (21) comporte une première arrivée de vapeur (22) reliée directement à la vanne d'arrêt (21) et une seconde arrivée de vapeur (23) mise en dérivation (28) sur la première arrivée de vapeur (22) et pourvue d'une électrovanne (31).

5. Autoclave à axe horizontal selon la revendication 4, caractérisé en ce que la première arrivée de vapeur (22) est située au-dessus du niveau (30) des condensats (29) et que la seconde arrivée de vapeur (23) est située au-dessous du niveau (30) des condensats (29) pour chauffer ces derniers.

6. Autoclave à axe horizontal selon les revendications 1 et 4, caractérisé en ce que le circuit de refroidissement comporte une rampe (55) pourvue d'arroseurs (56, 57, 58 ...) à jets circonscrits au-dessus de paniers (10, 11, 12) contenant les récipients à stériliser et alimentée, par l'intermédiaire d'une pompe multifonctions (43), en condensats (29) réchauffés par la seconde arrivée de vapeur (23) ou un mélange formé desdits condensats (29) et une arrivée de l'eau froide (45).

7. Autoclave à axe horizontal selon la revendication 6, caractérisé en ce que l'arrivée d'eau froide (45) comporte des moyens pour réguler avec précision la descente de température du mélange condensats (29) — eau froide (45) soit dégressive en continu, soit par paliers et constitués par une électrovanne (46) controlant le débit de l'eau froide et commandée à partir d'une sonde de température gérée par la programmation de l'autoclave (1).

8. Autoclave à axe horizontal selon la revendication 6, caractérisé en ce que le circuit de refroidissement comporte une vanne à trois voies (51) pour alimenter directement la rampe (55) par l'intermédiaire d'une conduite (54) ou indirectement cette rampe (55) par l'intermédiaire de l'échangeur de chaleur (60) récupérant les calories des condensats chauffés (29).

9. Autoclave à axe horizontal selon la revendication 8, caractérisé en ce que l'échangeur de chaleur (60) comporte une alimentation en fluide frigorifique pour abaisser la température de l'enceinte (3) à une température correspondant à celle régnant dans une armoire frigorifique.

**Patentansprüche**

1. Horizontalachsen-Autoklav zum Sterilisieren durch Dampfeinspritzung, umfassend einen Raum (3), der versehen ist mit einer dichten Tür (8), einer Dampfzufuhr (20, 22, 23), einer Preßluftzufuhr (32, 34, 36, 64), einem Motor (39)— Gebläse (38)-Aggregat zum Beschleunigen der Luft-Dampfmischung, einem Ablaß (40) mit einem Sicherheitsventil (41) zum Hinaustreiben der Raumluft, einem Auslaß (42) für die Kondensate (29), der verbunden ist mit einem geschlossenen Kühlkreis (43, 49, 54, 55, 56, 57, 58), der mit einer auswendigen Kaltwasserzufuhr (45) und einem Wärmeaustauscher (60) zum Rückgewinnen der Kalorien der Kondensate (29) und zum Beschleunigen des Kühlprozeßes versehen ist, dadurch gekennzeichnet, daß er einen im Raum (3) untergebrachten Preßluftkreis (34, 35, 36, 37, 64) umfaßt, dessen Preßluft, die ab dem Beginn der Sterilisierung durch den genannten Kreis (34, 35, 36, 37, 64) fließt, durch die im Raum (3) herrschende Temperatur erhitzt, danach in das Volumen des Raumes (3) eingespritzt wird.

2. Horizontalachsen-Autoklav nach Anspruch 1, dadurch gekennzeichnet, daß der im Raum (3) untergebrachte Preßluftkreis eine der Innenwand des Raumes (3) entlang, in der Längsrichtung angeordnete Rohrschlange (34) umfaßt, deren eines Ende (35) mit einem in der Preßluftzufuhrleitung (32) befindlichen Absperrventil (33) verbunden ist und deren anderes Ende (36), das, durch den Boden (5) desselben hindurch, außerhalb des Raumes (3) endet, einen mit einem Steuerelektroventil (37) versehenen, umgebogenen Teil (64) umfaßt und aufneu in den Raum (3) hineinführt.

3. Horizontalachsen-Autoklav nach Anspruch 2, dadurch gekennzeichnet, daß die Rohrschlange (34) ein Preßluftvolumen umfaßt, das proportional zum Volumen des Raumes (3) ist und dessen Flußmenge der in den Raum (3) hineingespritzten Preßluft von einem Temperaturmeßkopf und einem Druckregler gesteuert wird, die im Raum (3) untergebracht sind und vollautomatische Programmierungsmittel, wie einen Mikroprozeßor oder einen Mikrocomputer, einschalten.

4. Horizontalachsen-Autoklav nach Anspruch 1, dadurch gekennzeichnet, daß die mit einem Absperrventil (21) versehene Dampfzufuhrleitung (20) eine erste, direkt mit dem Absperrventil (21) verbundene Dampfzufuhr (22) und eine zweite, in By-pass (28) auf der ersten Dampfzufuhr (22) angebrachte und mit einem Elektroventil (31) versehene Dampfzufuhr (23) umfaßt.

5. Horizontalachsen-Autoklav nach Anspruch 4, dadurch gekennzeichnet, daß sich die erste Dampfzufuhr (22) oberhalb des Pegels (30) der Kondensate (29) und die zweite Dampfzufuhr (23) unterhalb des Pegels (30) der Kondensate (29) befinden, um dieselben zu erhitzen.

6. Horizontalachsen-Autoklav nach Ansprüchen 1 und 4, dadurch gekennzeichnet, daß der Kühlkreis eine Rampe (55) umfaßt, die mit umschriebenen Strahlinjektoren (55, 57, 58 ...) oberhalb der die zu sterilisierenden Gefäße enthaltenden Körbe (10, 11, 12) versehen ist und durch eine Multifunktionspumpe (43) mit von der zweiten Dampfzufuhr (23) erhitzten Kondensaten (29) oder mit einer aus der genannten Kondensaten (29) und einer Kaltwasserzufuhr (45) gebildeten Mischung gespeist wird.

7. Horizontalachsen-Autoklav nach Anspruch 6, dadurch gekennzeichnet, daß die Kaltwasserzufuhr (45) Mittel umfaßt für die genaue Regulierung der Temperatursenkung der Kondensaten (29) — Kaltwasser (45)-Mischung, entweder stufenlos oder stufenweise senkend, die bestehen aus einem Elektroventil (46), das die Kaltwasserflußmenge regelt und ab einem von der Programmierung des Autoklavs (1) gesteuerten Temperaturmeßkopf betätigt wird.

8. Horizontalachsen-Autoklav nach Anspruch 6, dadurch gekennzeichnet, daß der Kühlkreis ein Dreiwege-Ventil (51) zum Speisen der Rampe (55) direkt über eine Leitung (54) oder dieser Rampe (55) indirekt über den Wärmeaustauscher (60), der die Kalorien der erhitzten Kondensate (29) zurückgewinnt.

9. Horizontalachsen-Autoklav nach Anspruch 8, dadurch gekennzeichnet, daß der Wärmeaustauscher (60) eine Kühlfluidumzufuhr umfaßt, um die Temperatur des Raumes (3) bis zu einer Temperatur, die derjenigen, die in einer Kühlschrank herrscht, entspricht, zu senken.

## Claims

1. Horizontal-axis autoclave for sterilizing by steam injection, comprising an enclosure (3) fitted with a tight door (8), a steam supply (20, 22, 23), a compressed-air supply (32, 34, 36, 64), a motor (39) — fan (38) unit for accelerating the air-steam mixing, a drain (40) with a safety valve (41) for expelling the ambient air, an outlet (42) for the condensates (29) connected to a closed cooling circuit (43, 49, 54, 55, 56, 57, 58) provided with an external cold-water supply (45) and a heat-exchanger (60) for recovering the calories from the condensates (29) and for accelerating the cooling process, characterized in that it comprises a compressed-air circuit (34, 35, 37, 64) accomodated in the enclosure (3) and the compressed air of which, flowing through said circuit (34, 35, 36, 37, 64) since the beginning of the sterilization, is heated by the temperature prevailing in the enclosure (3), then injected into the volume of the enclosure (3).

2. Horizontal-axis autoclave according to claim 1, characterized in that the compressed-air circuit accomodated in the enclosure (3) comprises a coiled pipe (34) longitudinally arranged along the inner wall of the enclosure (3) one end (35) of which is connected to a checking valve (33) located on the compressed-air supply conduit (32) and the other end of which (36) ending outside the enclosure (3), passing through the bottom (5) of same, comprises a bend (64) provided with a control electrovalve (37) and is re-inserted into the enclosure (3).

3. Horizontal-axis autoclave according to claim 2, characterized in that the coiled pipe (34) comprises a compressed-air volume proportional to the volume of the enclosure (3) and the flow rate of the compressed air of which, injected into the enclosure (3), is controlled by a temperature probe and a pressure controller accomodated in the enclosure (3) and activating fully automatic programming means such as a microprocessor or a micro-computer.

4. Horizontal-axis autoclave according to claim 1, characterized in that the steam supply (20) provided with a checking valve (21) comprises a first steam-supply pipe (22) directly connected to the checking valve (21) and a second steam-supply pipe (23) placed in by-pass (28) on the first steam-supply pipe (22) and provided with an electrovalve (31).

5. Horizontal-axis autoclave according to claim 4, characterized in that the first steam-supply pipe (22) is located above the level (30) of the condensates (29) and that the second steam-supply pipe (23) is located under the level (30) of the condensates (29) in order to heat same.

6. Horizontal-axis autoclave according to claims 1 and 4, characterized in that the cooling circuit comprises a ramp (55) provided with circumscribed-jet sprinklers (55, 57, 58 ...) above the baskets (10, 11, 12) containing the receptacles to be sterilized and fed, through a multifunction pump (43), with condensates (29) heated by the second steam supply (23) or with a mixture formed by said condensates (29) and a cold-water supply (45).

7. Horizontal-axis autoclave according to claim 6, characterized in that the cold-water supply (45) comprises means for accurately regulating the temperature lowering of the mixture of condensates (29) — cold water (45), either continuously decreasing or stepwise and comprised of an electrovalve (46) controlling the cold-water flow rate and actuated from a temperature probe controlled by the programming of the autoclave (1).

8. Horizontal-axis autoclave according to claim 6, characterized in that the cooling circuit comprises a three-way-valve (51) for feeding the ramp (55) directly through a conduit (54) or this ramp (55) indirectly through the heat-exchanger (60) recovering the calories from the heated condensates (29).

9. Horizontal-axis autoclave according to claim 8, characterized in that the heat-exchanger (60) comprises a refrigerating-fluid feeding to lower the temperature of the enclosure (3) to a temperature corresponding to the one prevailing in an ice-box.

FIG. 1

FIG. 2